# FASCICULE DE BREVET EUROPEEN

(11) **EP 2 874 618 B1**
(45) Date de publication et mention de la délivrance du brevet: **19.02.2020**
(21) Numéro de dépôt: 13744754.6
(22) Date de dépôt: 16.07.2013
(51) Int. Cl.: A61K 31/167, A61K 31/706, G01N 33/50

(54) **UTILISATION DE COMPOSES MODIFIANT L'EPIGENOME POUR LE TRAITEMENT DES MALADIES GENETIQUES MUSCULAIRES LIEES A UNE ANOMALIE DE CONFORMATION PROTEIQUE**
VERWENDUNG EPIGENOMMODIFIZIERENDER VERBINDUNGEN ZUR BEHANDLUNG VON GENETISCHEN MUSKELERKRANKUNGEN IN ZUSAMMENHANG MIT EINER PROTEINFEHLFALTUNGSERKRANKUNG
USE OF EPIGENOME-MODIFYING COMPOUNDS FOR THE TREATMENT OF GENETIC MUSCULAR DISEASES LINKED TO A PROTEIN-CONFORMATIONAL DISORDER

(30) Priorité: 19.07.2012 FR 1257021
(43) Date de publication de la demande: 27.05.2015
(73) Titulaire: Genethon, 91002 Evry (FR)
(72) Inventeur: RICHARD, Isabelle, F-91100 Corbeil Essonnes (FR)
(74) Mandataire: Cabinet Laurent & Charras
(86) Numéro de dépôt international: PCT/FR2013/051705
(87) Numéro de publication internationale: WO 2014/013184

(56) Documents cités:
- WO-A1-2004/050076
- WO-A1-2011/068522
- WO-A2-00/20582
- WO-A2-03/066885
- WO-A2-2007/014327
- WO-A2-2008/009802
- E. FUJITA ET AL: "Two endoplasmic reticulum-associated degradation (ERAD) systems for the novel variant of the mutant dysferlin: ubiquitin/proteasome ERAD(I) and autophagy/lysosome ERAD(II)", HUMAN MOLECULAR GENETICS, vol. 16, no. 6, 2006, pages 618-629, XP055052681, ISSN: 0964-6906, DOI: 10.1093/hmg/ddm002
- DERRICK SEK TONG ONG ET AL: "Chemical and/or biological therapeutic strategies to ameliorate protein misfolding diseases", CURRENT OPINION IN CELL BIOLOGY, vol. 23, no. 2, 2011, pages 231-238, XP028193846, ISSN: 0955-0674, DOI: 10.1016/J.CEB.2010.11.002 [extrait le 2010-11-20]
- TAYEBEH SOHEILI ET AL: "Rescue of sarcoglycan mutations by inhibition of endoplasmic reticulum quality control is associated with minimal structural modifications", HUMAN MUTATION, vol. 33, no. 2, février 2012 (2012-02), pages 429-439, XP055052557, ISSN: 1059-7794, DOI: 10.1002/humu.21659
- ALEKSEY G. KAZANTSEV ET AL: "Therapeutic application of histone deacetylase inhibitors for central nervous system disorders", NATURE REVIEWS DRUG DISCOVERY, vol. 7, no. 10, octobre 2008 (2008-10), pages 854-868, XP055044174, ISSN: 1474-1776, DOI: 10.1038/nrd2681

## Description

### DOMAINE DE L'INVENTION

La présente demande concerne le traitement des maladies génétiques musculaires causées par un défaut de conformation protéique et dans lesquelles la protéine présentant le défaut de conformation subit une dégradation cellulaire. Des maladies particulièrement visées sont par exemple les sarcoglycanopathies, les dysferlinopathies, les anoctaminopathies (liées à un défaut de l'anoctamine 5) et les dystrophies associées à une anomalie de FKRP (pour « Fukutin-Related Protein »).

Plus précisément, elle décrit l'identification et l'utilisation d'agents modifiant l'épigénome comme médicaments pour le traitement de ces maladies.

### ETAT DE LA TECHNIQUE

Les pathologies dues à une anomalie des fibres musculaires sont regroupées sous le terme de myopathies et sont caractérisées par une déstabilisation et une dégénérescence musculaire conduisant le plus souvent à une altération et à une perte des fonctions locomotrices.

Plus de 80 myopathies rares d'origine génétique ont été identifiées. Elles peuvent avoir une transmission dominante, récessive ou liée au chromosome X. La prévalence de chacune des formes est faible (de 1/200000 à 1/8500), même si la fréquence cumulée atteint environ 1/3000.

Parmi ces pathologies, le groupe le plus complexe est celui des dystrophies musculaires progressives qui comprend au moins 25 pathologies différentes qui peuvent être divisées en dystrophies musculaires proximales, dites dystrophies des ceintures, et en dystrophies musculaires distales.

Les dystrophies musculaires proximales sont caractérisées par une atrophie progressive et sélective de certains muscles des ceintures scapulaires et pelviennes et les dystrophies musculaires distales par une atteinte initiale des muscles des mains, des pieds, des avant-bras ou des jambes.

Le défaut génétique conduit à un processus cyclique de dégénérescence / régénération du tissu musculaire conduisant à un remaniement pathologique de celui-ci. Sur le plan histologique, ce processus est caractérisé par des fibres possédant un noyau centralisé, témoignant des étapes de nécrose et régénération, des infiltrations macrophagiques et adipeux et une fibrose secondaire.

Les symptômes cliniques peuvent être très hétérogènes selon le type de pathologie, allant de la simple fatigue jusqu'à la perte de la marche, une diminution de force qui s'accompagne habituellement d'une atrophie musculaire **(Daniele *et al.,*** 2007). Ces pathologies peuvent donc avoir un fort impact sur la qualité de vie des patients en raison de la perte musculaire pouvant conduire au décès par atteinte respiratoire ou cardiaque.

De manière plus précise, les dystrophies musculaires proximales, ou dystrophies des ceintures (« Limb Girdle Muscular Dystrophy » ou LGMD), sont classées en fonction de leur mode de transmission : LGMD1 pour les formes dominantes et LGMD2 pour les formes récessives **(Bushby and Beckmann,** 1995).

Les caractéristiques communes des LGMD1 sont : une apparition des symptômes à l'âge jeune-adulte ou adulte, une évolution lente, une faible fréquence de perte de la marche et des valeurs de créatine kinase relativement normales. Ces formes dominantes sont moins fréquentes que les formes récessives, représentant moins de 10% de toutes les LGMDs **(Nigro,** 2003) avec seulement une ou quelques familles rapportées pour chaque type.

A ce jour, seize formes de dystrophies des ceintures autosomiques récessives (LGMD2) ont été identifiées et annotées par l'addition d'une lettre capitale (LGMD2A à 2P). Tous les gènes responsables de ces dystrophies ont été identifiés. Les protéines correspondantes ont des localisations et des rôles variés.

De manière encore plus complexe, il faut noter que des mutations dans un certain nombre de gènes impliqués dans les LGMD2 sont également associées à une présentation clinique de type distal. A titre d'exemples, on peut citer :
- la dysferline qui, mutée, peut également conduire à une myopathie de Miyoshi ;
- l'anoctamine 5 qui, mutée, peut également conduire à une myopathie de type Miyoshi like type 3 ;
- la titine qui, mutée, peut également conduire à une dystrophie musculaire tibiale (TMD).

Parmi les LGMD2, les sarcoglycanopathies sont des maladies génétiques dues à des mutations dans les gènes des sarcoglycanes. Ces protéines, au nombre de 4, font partie d'un complexe associé à la dystrophine présent à la membrane de la cellule musculaire et qui joue un rôle essentiel dans la protection de la fibre musculaire au cours des contractions.

Ainsi, les formes suivantes de sarcoglycanopathies ont été répertoriées :
- la LGMD2C, provoquée par une mutation du gène codant la γ-sarcoglycane **(Ben Othmane *et al.,*** 1995) ;
- la LGMD2D, provoquée par une mutation du gène codant l'a-sarcoglycane **(Passos-Bueno** ***et al.**,* 1993) ;
- la LGMD2E provoquée par une mutation du gène codant la β-sarcoglycane (**Lim** ***et al.**,* 1995) ; et
- la LGMD2F, provoquée par une mutation du gène codant la δ-sarcoglycane **(Passos-Bueno** ***et al.**,* 1996).

Des mutations responsables dans chacun de ces gènes ont pu être identifiées : Ainsi, la mutation la plus fréquente dans la LGMD2D est la substitution de l'arginine en position 77 par une cystéine (R77C). Une mutation spécifique dans le gène codant pour la γ-SG, la C283Y, à l'origine de LGMD2C, est fréquemment retrouvée dans la communauté Tzigane en Europe **(Piccolo *et al.,*** 1996; **Todorova *et al.,*** 1999). Une autre mutation dans le gène de la γ-SG, la del521T, est presque exclusivement retrouvée dans les familles tunisiennes **(Kefi, *et al.,*** 2003). Les patients de la communauté Amish aux Etats-Unis, où la LGMD2E est fréquente, sont porteurs de la mutation T151R **(Duclos,** ***et al.**,* 1998; **Lim** ***et al.**,* 1995).

En tout état de cause, il n'existe pas actuellement de traitement curatif vraiment efficace de ces maladies. Bien que la kinésithérapie soit un élément de confort très apprécié par ces patients, leur prise en charge consiste principalement en divers moyens médicamenteux et/ou techniques visant à améliorer la qualité de vie des patients.

En rapport avec le traitement des sarcoglycanopathies, le document WO 2008/009802 a préconisé l'utilisation d'inhibiteurs des α-mannosidases de classe I. Dans ce cadre, il a été montré qu'un certain nombre de mutations dans les gènes des sarcoglycanes conduisaient, non pas à une perte de fonction de la protéine, mais à une dégradation de la protéine anormalement conformée par le système de contrôle-qualité de la cellule. Ainsi, les inhibiteurs préconisés, dirigés contre ce système, empêcheraient la dégradation de la protéine mutée, qui retrouve alors une localisation membranaire fonctionnelle.

Le document WO 2004/050076, quant à lui, a préconisé l'expression d'un gène homologue silencieux à l'aide d'inhibiteur des histones désacélylases, pour traiter des dystrophies musculaires causées par la déficience d'un gène adulte.

Il existe toutefois un besoin évident de développer de nouvelles approches thérapeutiques pour prendre en charge les maladies génétiques musculaires telles que les sarcoglycanopathies.

### EXPOSE DE L'INVENTION

Ainsi, c'est une toute nouvelle approche thérapeutique qui est proposée dans le cadre de la présente invention. En effet, le Demandeur a montré qu'il était possible de restaurer la fonctionnalité d'une protéine musculaire présentant un défaut de conformation, à savoir un mauvais repliement, à l'aide d'un composé modifiant l'épigénome.

Plus précisément et selon un premier aspect, la présente invention concerne donc une composition pharmaceutique comprenant au moins un composé choisi parmi :
- un inhibiteur de la méthylation de l'ADN, avantageusement un inhibiteur des méthyltransférases ; ou
- un inhibiteur des histones désacétylases,
pour utilisation dans le traitement des maladies génétiques musculaires causées par une anomalie de conformation d'au moins une protéine, ladite anomalie entraînant la dégradation cellulaire de la protéine, la maladie génétique musculaire étant une dystrophie musculaire progressive, la protéine étant choisie dans le groupe constitué des sarcoglycanes, de la dysferline, de l'anoctamine 5 et de la protéine FKRP (« Fukutin-Related Protein »).

En d'autres termes, il est décrit d'utiliser une composition comprenant un composé modifiant l'épigénome pour préparer un médicament destiné au traitement des maladies génétiques musculaires causées par une anomalie de conformation d'au moins une protéine entraînant sa dégradation cellulaire. La demande décrit donc une méthode de traitement de maladies génétiques musculaires causées par une anomalie de conformation d'au moins une protéine entraînant sa dégradation cellulaire comprenant l'administration, à une dose effective, d'une composition comprenant un composé modifiant l'épigénome.

Ladite composition peut en outre contenir tout composé ou excipient acceptable, notamment pharmaceutiquement. Elle peut en outre comprendre d'autres principes actifs destinés à traiter la même pathologie ou une autre pathologie. Selon un mode de réalisation particulier, le ou les composé(s) modifiant l'épigénome sont les seuls principes actifs de la composition.

Il peut également être envisagé d'associer, dans la même composition, au moins deux composés modifiant l'épigénome de nature distincte, pour une administration simultanée ou décalée dans le temps.

La voie d'administration peut aussi bien être intramusculaire, loco-régionale, ou intraveineuse, voire sous-cutanée, intrapéritonéale ou orale.

Le mode d'administration, la dose administrée ainsi que la fréquence d'administration sont déterminés au cas par cas, selon des protocoles classiques connus de l'homme du métier.

On entend par « épigénome » l'ensemble des signaux biochimiques transmissibles et potentiellement réversibles qui sont associés aux gènes afin d'en gouverner l'expression et d'assurer ainsi l'exécution du programme génétique lié à l'activation et à l'inactivation de ces gènes sans modification de séquence de l'ADN d'une cellule différenciée donnée.

Ainsi, la séquence d'ADN qui compose les gènes est identique dans toutes les cellules d'un même individu mais les protéines codées par ces gènes pourront être produites à des moments ou à des endroits différents suivant les marques épigénétiques qui sont présentes sur les gènes.

Le support biochimique de l'épigénome est multiple et complexe et implique :
- des modifications au niveau de l'ADN ;
- des modifications au niveau des histones ;
- la participation de certaines protéines, telles que les polycombs ;
- la participation d'ARN non codants, de petite taille (les microARN) et de grande taille (les longs ARN non-codants).

En d'autres termes, un composé modifiant l'épigénome décrit dans le cadre de la demande est un modulateur d'un effecteur épigénétique, c'est-à-dire capable d'en moduler au moins partiellement l'expression et/ou l'activité. On entend par « modulateur » aussi bien un activateur (ou inducteur) qu'un inhibiteur (ou suppresseur).

Ces composés peuvent être de toute nature, par exemple des protéines, des peptides, des anticorps, des molécules chimiques ou des acides nucléiques (oligonucléotides antisens, ARNsi, ARNsh, ribozymes, ...).

Selon un premier mode de réalisation, c'est le niveau d'expression et/ou de production de l'effecteur épigénétique qui est modulé (diminué ou augmenté) par le composé en question. Ceci peut être aisément testé par l'homme du métier, notamment grâce aux techniques suivantes :
- niveau d'expression du transcrit par Northern blot ou PCR ;
- niveau de production de la protéine par détection à l'aide d'anticorps appropriés (Western blot ou ELISA).

Selon un autre mode de réalisation, l'effecteur épigénétique est produit et c'est son activité qui est modulée (augmentée ou diminuée) par le composé en présence. Dans ce cas de figure, cette activité est quantifiée à l'aide d'un test approprié en fonction de l'effecteur.

Concernant la nature des composés modifiant l'épigénome, il est connu que le premier niveau du contrôle épigénétique concerne la méthylation de l'ADN qui se produit au niveau des dinucléotides CpG et se met en place très tôt au cours du développement. Elle résulte notamment de l'action concertée de trois méthyltransférases de l'ADN (ou DNMT).

Ainsi, selon un aspect particulier, le composé modifiant l'épigénome peut être un inhibiteur de la méthylation de l'ADN, notamment un inhibiteur des méthyltransférases de l'ADN. Cette classe de molécules est particulièrement adaptée pour les applications *in vitro.*

Dans cette catégorie, on peut notamment citer :
- des analogues nucléosides : ils sont incorporés dans l'ADN en réplication. Ils inhibent ainsi la méthylation de l'ADN et réactivent des gènes silencieux. Il peut s'agir de :
   ∘ la 5-azactidine (ou 5-aza), de formule : 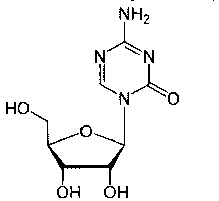 la 5-aza-2'-déoxctidine ou décitabine, de formule : 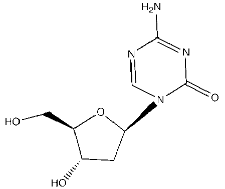
   ∘ la zébularine ;
- des analogues non nucléosidiques : ils inhibent les DNMT en bloquant le site actif de l'enzyme ou en se fixant sur les régions CpG de l'ADN empêchant ainsi la liaison des DNMT à l'ADN. Il peut s'agir de :
   ∘ la procainamide ;
   ∘ la procaine ;
   ∘ l'epigallocatechin-3 gallate (EGCG) ;
- des oligonucléotides antisens :
   ∘ DNMT1 ASO ;
- l'hydralazine, de formule : 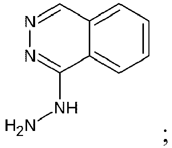
- le SGI110 (S110), de formule :

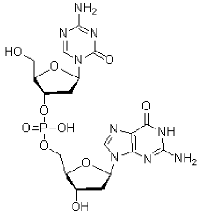

Alternativement et de manière préférentielle pour les applications *in vivo,* le composé modifiant l'épigénome est un inducteur ou activateur de la déméthylation de l'ADN.

De manière générale, la capacité d'un composé à moduler la méthylation de l'ADN peut être aisément testée : Le profil de méthylation de l'ADN peut être déterminé grâce à un traitement de l'ADN au bisulfite, qui convertit les cytosines non méthylées en uracile mais n'affecte pas les méthylcytosines. Différentes méthodes basées sur le séquençage direct, le pyroséquençage, la PCR ou l'hybridation peuvent ensuite être utilisées pour différencier les allèles.

Ainsi, grâce à ce test simple, des inhibiteurs de la méthylation de l'ADN, susceptibles d'être utilisés dans le cadre de la présente invention, peuvent être aisément identifiés.

Un autre niveau d'action concerne les modifications covalentes des histones. De manière préférentielle, ces modifications concernent : l'acétylation des résidus lysine, la méthylation des résidus lysine et des arginine, la phosphorylation des résidus thréonine et sérine, l'ubiquitination et la sumoylation des résidus lysine.

Or, ces modifications au niveau des histones peuvent aussi bien avoir un effet positif que négatif sur l'expression des gènes. Le composé d'intérêt peut aussi bien être de type activateur qu'inhibiteur.

En outre, il est possible d'agir sur les enzymes de modification des histones soit directement au niveau de leur activité, soit génétiquement au niveau de leur expression.

En pratique, il est possible d'analyser les modifications post-transcriptionnelles des histones, et donc d'évaluer l'impact d'un composé d'intérêt, par les techniques bien connues de Westen Blot ou d'ELISA, mettant en œuvre une batterie d'anticorps spécifiques des modifications des histones (**Egelhofer *et al.,*** 2011).

A titre d'exemple et comme déjà dit, il est possible de moduler le niveau d'acétylation des histones. Or celui-ci est la résultante de l'activité de deux enzymes antagonistes : les histones désacétylases (HDAC), conduisant à une chromatine réprimée et les histone-acétyltransférases (HAT) qui permettent l'expression génique.

Ainsi et selon un autre aspect de l'invention, le composé modifiant l'épigénome peut être un inhibiteur des histones désacétylases (HDAC).

A noter qu'il existe plusieurs classes d'inhibiteurs des HDACs en fonction de leur mode d'inhibition et de la catégorie des HDACs qu'ils ciblent. Parmi les inhibiteurs de la désacétylation des histones, on peut citer :
∘ les acides hydroxamiques ou leurs sels :
   ▪ trichostatine A (TSA) ;
   ▪ apha compound 8 ;
   ▪ MC1568 ;
   ▪ Tubucine ;
   ▪ suberoylanilide hydroxamic acid (SAHA ou vorinostat), de formule : 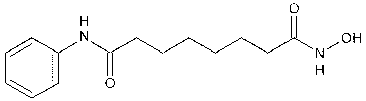
∘ les tétrapeptides cycliques et des depsipeptides :
   ▪ trapoxine B ;
   ▪ apicidine ;
∘ les benzamides :
   ▪ entinostat (MS-275), de formule : 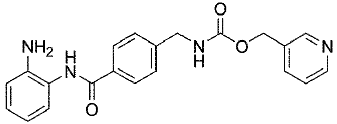
   ▪ CI-994 ;
   ▪ 106
   ▪ Mocetinostat (MGCD0103)
∘ les cétones électrophiles :
   ▪ les cétones trifluorométhylées ;
   ▪ les α-cétoamides ;
∘ les composés acides aliphatiques :
   ▪ le phénylbutyrate, de formule : 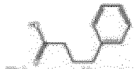
   ▪ l'acide valproïque ;
∘ belinostat (PXD101) ;
∘ LAQ 824 ;
∘ Panobinostat (LBH589) ;
   - les autres molécules :
      ∘ nicotinamide ;
      ∘ dihydrocoumarine ;
      ∘ naphthopyranone ;
      ∘ 2-hydroxynaphaldéhydes ;
      ∘ 10-hydroxy-2-decenoic acid (10HDA) ;
      ∘ Abexinostat (PCI-24781) ;
      ∘ SB939 ;
      ∘ Resminostat (4SC-201) ;
      ∘ Givinostat (ITF2357) ;
      ∘ CUDC-101 ;
      ∘ AR-42 ;
      ∘ CHR-2845 ;
      ∘ CHR-3996 ;
      ∘ 4SC-202 ;
      ∘ CG200745 ;
      ∘ ACY-1215 ;
      ∘ Sulforaphane ;
      ∘ Kevetrine ;
      ∘ Apicidin ;
      ∘ Sodium butyrate ;
      ∘ (-)-Depudecin ;
      ∘ Sirtinol ;
      ∘ *N*-Hydroxy-1,3-dioxo-1*H*-benz[de]isoquinoline-2(3*H*)-hexanamide ou Scriptaid ;
      ∘ Le dérivé hydroxamate de l'acide butyrique ;
      ∘ L'isobutyramide ;
      ∘ Le CBHA (m-carboxycinnamic acid bishydoxyamide) ;
      ∘ L'HC toxin ;
      ∘ Le M344 (4-diméthylamino-N-(6-hydroxycarbamoyl-hexyl)-benzamide) ;
      ∘ Le Nullscript (4-(1,3-dioxo-1H,3H-benzo[de]isoquinolin-2-yl)-N-hydroxybutanamide) ;
      ∘ PCI-34051, de formule :

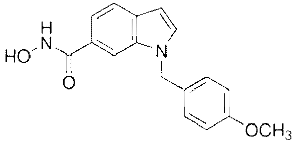

Les formules chimiques d'un certain nombre de ces inhibiteurs sont décrites dans le document Kazantsev and Thompson (Nature Reviews Drug Discovery, vol. 7, n° 10, 2008, 854-866). Par ailleurs, une méthode de criblage d'inhibiteurs des HDAC est par exemple décrite dans le document WO 03/066885.

Avantageusement et pour cette classe de composés, l'inhibiteur utilisé est le SAHA.

En outre, des inhibiteurs de la méthylation des histones peuvent être :
- le SL11144, de formule : 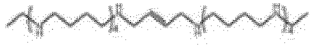
- le DZNep (3-Deazaneplanocin : inhibiteur de la S-adénosylhomocystéine hydrolase), de formule :

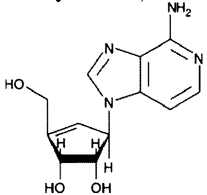

Un troisième niveau de contrôle peut être exercé au niveau du complexe PRC. En effet, les protéines du groupe polycomb (PcG) sont des facteurs chromatiniens connus pour maintenir l'état transcriptionnel réprimé de leurs gènes cibles au cours du développement. Ces facteurs agissent sous forme de larges complexes multimériques appelés *Polycomb Repressive Complex* (PRC) qui se fixent sur l'ADN, au niveau de séquences régulatrices appelées *PcG response elements* (PRE) afin de réprimer des gènes par méthylation. En effet, les complexes PRC contiennent également une activité histone méthyltransférase (HMTase) intrinsèque et répriment l'expression par la méthylation des histones.

Les protéines polycomb peuvent par exemple être ciblées, notamment inhibées ou inactivées au niveau de leur expression à l'aide d'un antisens ou en réalisant du saut d'exons. L'expression ou l'activité des protéines polycomb peut être suivie par les techniques classiques suivantes : niveau d'expression par Westen Blot ; liaison à l'ADN par gels retards (« gel mobility shift ») ou précipitation de la chromatine.

Enfin, l'implication dans le contrôle de l'expression des gènes des ARN non codants, les microARN et les longs ARN non codants, a été soulignée.

Les microARN (miARN) sont une classe d'ARN non-codants d'environ 22 nucléotides qui modulent négativement l'expression des gènes de façon post-transcriptionnelle. En se fixant par complémentarité sur l'ARN cible, ils vont conduire soit à la dégradation de cet ARN soit en inhiber la traduction. Les miARN peuvent, de plus, influencer les mécanismes épigénétiques en contrôlant la méthylation de l'ADN et les modifications des histones par recrutement de complexes au niveau du transcrit naissant correspondant.

Un autre type d'acteur en épigénétique sont les longs ARN non codants (ARNlnc). Ils sont transcrits au niveau de séquences géniques ou intergéniques et n'ont pas ou peu de cadres de lecture ouverts. Ces ARN peuvent cibler la chromatine ou différents aspects de la transcription en s'associant avec différents facteurs (activateurs de transcription ou répresseurs) ainsi que déstabiliser ou au contraire stabiliser un ARN codant dans le cytoplasme.

A noter que de manière connue, l'effet d'un composé sur l'expression d'un ARN non codant peut être aisément analysé, par exemple par Northern Blot ou par RT-PCR quantitative.

Ainsi, le composé modifiant l'épigénome mis en œuvre dans le cadre de l'invention est un inhibiteur :
- de la méthylation de l'ADN ; ou
- des histones désacétylases;

Un certain nombre de composés actuellement disponibles sont listés ci-dessus, notamment le SAHA. Toutefois, cette liste ne peut se prétendre être exhaustive. Ainsi, tout composé pouvant moduler l'épigénome, via les mécanismes mentionnés ci-dessus, peut être utilisé dans le cadre de l'invention. Avantageusement, il s'agit du phénylbutyrate, du SAHA ou de la 5-azacytidine.

Comme déjà dit, la présente invention s'inscrit dans le traitement des maladies génétiques musculaires causées par une anomalie de conformation d'au moins une protéine entraînant sa dégradation cellulaire.

Les maladies génétiques sont, par définition, des maladies résultant d'une ou plusieurs mutations dans un ou plusieurs gènes.

Avantageusement, sont visées dans la présente demande les maladies monogéniques, c'est-à-dire lié à un seul gène, en l'occurrence celui codant la protéine présentant une anomalie de conformation.

La ou les mutations responsables de l'anomalie de conformation de la protéine résultante peuvent être des mutations ponctuelles. Toutefois, le défaut de conformation peut être lié à des mutations non strictement ponctuelles, par exemple la délétion d'un codon dans le gène qui code une protéine toujours active si elle n'est pas dégradée.

Plus précisément, la présente demande vise les pathologies musculaires récessives.

Comme déjà dit, la présente demande vise les pathologies du muscle, avantageusement celles affectant les muscles squelettiques mais aussi le muscle cardiaque, avantageusement les muscles squelettiques.

Dans le cadre de l'invention, on entend par « anomalie de conformation d'au moins une protéine » ou « anomalie de conformation protéique » le fait que la protéine causatrice de la maladie présente un défaut de repliement, lié à la présence d'au moins une mutation dans le gène la codant, ladite anomalie entraînant la dégradation de ladite protéine par la cellule. Les pathologies visées sont donc aisément identifiées, par exemple à l'aide d'anticorps dirigées contre la protéine mutée. En effet, même si celle-ci est correctement exprimée (ce qui peut être vérifié au niveau des transcrits), elle n'est pas détectée à l'aide d'un tel anticorps, puisque dégradée.

Dans le cadre de l'invention, il a été mis en évidence que la dégradation par les cellules musculaires des protéines mal conformées était contrôlée par des mécanismes épigénétiques. Ainsi, la solution proposée repose sur l'utilisation de composés modifiant l'epigénome afin d'éviter la dégradation des protéines mutées, d'assurer leur adressage dans leur compartiment cellulaire final et ainsi de restaurer un phénotype normal.

Selon un aspect particulier, la protéine présentant l'anomalie de conformation et subissant la dégradation cellulaire est une protéine membranaire ou associée à la membrane, éventuellement intégrée dans un complexe protéique.

Selon l'invention, la maladie génétique musculaire liée à une anomalie de conformation protéique est une dystrophie musculaire progressive, aussi bien de type proximal que distal.

Parmi les dystrophies musculaires progressives, des maladies privilégiées sont :
- les sarcoglycanopathies, à savoir l'a-sarcoglycanopathie ou LGMD2D liée à un défaut de l'a-sarcoglycane, la β- sarcoglycanopathie ou LGMD2E liée à un défaut de la β-sarcoglycane, la γ-sarcoglycanopathie ou LGMD2C liée à un défaut de la γ-sarcoglycane, ou la δ-sarcoglycanopathie ou LGMD2F liée à un défaut de la δ-sarcoglycane ;
- les dysferlinopathies (LGMD2B ou myopathie de Miyoshi) liées à un défaut de la dysferline ;
- les anoctaminopathies (LGMD2L ou myopathie de Miyoshi type 3) liées à un défaut de l'anoctamine 5 ;
- la myopathie des ceintures avec déficit en FKRP ou LGMD2I, liée à un défaut de la protéine FKRP (pour « fukutin-related protein »).

Plus généralement, les maladies décrites sont choisies dans le groupe suivant :
- des dystrophies musculaires progressives :
   ∘ impliquant la dysferline (DYSF)
   ∘ impliquant la γ-sarcoglycane
   ∘ impliquant l'a-sarcoglycane
   ∘ impliquant la β-sarcoglycane
   ∘ impliquant la δ-sarcoglycane
   ∘ impliquant l'anoctamine 5 (Ano5)
   ∘ impliquant la fukutin related protein (FKRP)
   ∘ impliquant la fukutine (FKTN)
   ∘ impliquant la protéine-O-mannosyltransférase 1 (POMT1)
   ∘ impliquant la protéine-O-mannosyltransférase 2 (POMT2)
   ∘ impliquant la protein-O-linked mannose β 1,2-acétylglucosaminyl-transférase (POMGT1)
   ∘ impliquant la cavéoline 3 (Cav3)
   ∘ impliquant l'UDP-N-acétyl glucosamine 2-épimérase (GNE)
- des dystrophies musculaires congénitales (MDC) :
   ∘ impliquant l'a-dystroglycane (DAG1)
   ∘ impliquant la laminine alpha2 (LAMA2)
   ∘ impliquant la like-glycosyltransférase (LARGE)
   ∘ impliquant la collagène 6A1, 6A2 ou 6A3
   ∘ impliquant la sélénoprotéine 1 (SEPN1)
   ∘ impliquant l'intégrine alpha7 (ITGA7)
   ∘ le récepteur à la ryanodine (RYR)
- d'autres maladies touchant le muscle squelettique ou cardiaque, associé éventuellement à des atteintes d'autres organes :
   ∘ la cardiomyopathie ventriculaire droite arrhythmogénique impliquant la protéine transmembranaire 43 (TMEM43)
   ∘ la liposdystrophie congénitale de type 4 avec dystrophie musculaire impliquant la polymérase I et le transcript release factor (PTRF)
   ∘ la Chondrodystrophic myotonia ou Syndrome de Schwartz Jampel impliquant l'heparan sulfate (HSPG2)
   ∘ la maladie de Danon impliquant la lysosomal-associated membrane protéine 2 (LAMP2)
   ∘ la Fibrodysplasia ossificans progressiva impliquant le type I activin A receptor (ACVR1).

Pour une même maladie, différentes mutations peuvent affecter le même gène et être responsable de la mauvaise configuration de la protéine codée. Ainsi et à titre d'exemples pour les sarcoglycanopathies, les principales mutations ponctuelles répertoriées à ce jour sont listées dans le document WO 2008/009802.

Dans le cadre de la présente demande, la faisabilité de l'invention a par exemple été démontrée en relation avec la mutation R77C de l'a-sarcoglycane. Les protéines portant une mutation E262K dans la sous-unité δ ou une mutation Q11E dans la sous-unité β peuvent également répondre favorablement au traitement préconisé.

De manière adaptée, la protéine mutée causatrice de la maladie n'est pas la dystrophine et la pathologie à traiter n'est pas la myopathie de Duchenne (DMD) ou de Becker (BMD), dans laquelle la dystrophine est tronquée, et non pas dégradée car mal repliée comme dans l'invention.

D'autres procédés ou utilisations peuvent découler de la présente demande :
Selon un autre aspect, la demande décrit un procédé d'identification ou d'évaluation de l'efficacité d'un composé modifiant l'épigénome qui comprend les étapes suivantes :
- mise en contact d'une cellule produisant une protéine mal repliée causant une maladie génétique musculaire, avec ledit composé ;
- détermination du taux de protéine correctement repliée, avantageusement par localisation de la protéine dans la cellule.

De manière avantageuse, le mauvais repliement de la protéine entraine sa dégradation par la cellule.

La production par la cellule de la protéine mal repliée causant une maladie génétique musculaire peut être obtenue par transfection de la cellule à l'aide du gène muté codant ladite protéine.

La protéine mal repliée causant une maladie génétique musculaire peut être une sarcoglycane causant une sarcoglycanopathie. Encore plus avantageusement, il s'agit d'une α-sarcoglycane, par exemple une α-sarcoglycane portant une mutation R77C. En tout état de cause, cette protéine peut être utilisée comme témoin positif dans le procédé décrit ci-dessus, dans la mesure où il a été démontré, dans la présente demande, que cette protéine mutée répondait favorablement à un composé modifiant l'épigénome.

Avantageusement, la mise en contact entre la cellule et le composé a lieu pendant plusieurs minutes, voire plusieurs heures.

Le taux de protéine correctement repliée est corrélé à l'efficacité du composé testé en tant que composé modifiant l'épigénome. Ce taux est avantageusement déterminé par détection, voire localisation, de la protéine ou du complexe protéique auquel elle appartient. De manière préférée, la détection de la protéine ou du complexe protéique est réalisée par immunohistochimie, à l'aide d'anticorps dirigé contre la protéine en question ou contre l'une des protéines du complexe protéique auquel elle appartient.

Alternativement, la protéine d'intérêt ou une des protéines du complexe d'intérêt peut être marquée, notamment par fluorescence, puis détectée par microscopie ou par triage. Cette solution est avantageuse dans la mesure où l'étape de détection est moins lourde que la détection immunologique. Toutefois, il est important de vérifier que la protéine fusion (protéine d'intérêt + marquage) ne perturbe pas la formation du complexe protéique.

Dans le cas où la protéine ou le complexe protéique est détecté, voire localisé dans son compartiment cellulaire final, il est conclu que le composé testé est un composé efficace pour la modification de l'épigénome.

De manière avantageuse, le procédé est mis en œuvre en parallèle :
- sur des cellules exprimant la protéine non mutée comme témoin positif de la localisation de la protéine ;
- en réalisant une incubation en l'absence du composé testé, ce témoin permettant de conclure quant à l'effet éventuel du composé testé.

Selon un autre aspect, la présente demande décrit un procédé d'identification d'un composé pour le traitement de maladies génétiques musculaires liées à une anomalie de conformation d'au moins une protéine entraînant sa dégradation cellulaire, consistant à évaluer le potentiel du composé à modifier l'épigénome.

Ainsi, la demande décrit également un procédé d'identification d'un composé pour le traitement de maladies génétiques musculaires liées à une anomalie de conformation d'au moins une protéine entraînant sa dégradation cellulaire, comprenant les étapes suivantes :
- mise en contact d'une cellule produisant une protéine mal repliée causant une maladie génétique musculaire, avec ledit composé ;
- détermination du taux de protéine correctement repliée.

Selon un autre aspect, la demande décrit un procédé d'identification de maladies génétiques musculaires liées à une anomalie de conformation d'au moins une protéine entraînant sa dégradation cellulaire, comprenant les étapes suivantes :
- mise en contact d'une cellule susceptible de produire une protéine mal repliée causant une maladie génétique musculaire, avec un composé modifiant l'épigénome ;
- détermination du taux de protéine correctement repliée.

Dans ce mode de réalisation, il s'agit de tester des cellules, avantageusement musculaires, d'un patient susceptible d'être atteint d'une maladie génétique musculaire liée à une anomalie de conformation d'au moins une protéine entraînant sa dégradation cellulaire. Alternativement, le gène du patient, suspecté de porter la ou les mutations, est transfecté dans une cellule pour réaliser le test *in vitro.*

Si le patient est effectivement atteint, il est attendu que la protéine présentant une anomalie de conformation ne soit pas détectable car dégradée. Ceci peut être aisément testé comme décrit ci-dessus, notamment à l'aide d'anticorps dirigés contre ladite protéine.

En présence du composé modifiant l'épigénome, il est attendu que le taux de protéine correctement repliée soit augmenté et que la protéine devienne détectable, notamment dans le test de détection mis en œuvre dans l'étape précédente.

Si, de fait, le taux de protéine correctement repliée augmente avec l'ajout du composé modifiant l'épigénome, il peut être conclu qu'il s'agit bien d'une maladie génétique musculaire liée à une anomalie de conformation de la protéine testée, entraînant sa dégradation cellulaire.

Plus généralement et selon un autre aspect, la demande décrit une méthode de production par une cellule d'une protéine, éventuellement recombinante, dans une configuration active consistant à mettre la cellule en contact avec un composé modifiant l'épigénome.

### EXEMPLES DE REALISATION

L'invention et les avantages qui en découlent ressortiront mieux des exemples de réalisation suivants, à l'appui des figures annexées. Ceux-ci n'ont toutefois aucune portée limitative.

L'invention est illustrée plus avant en rapport avec l'a-sarcoglycanopathie liée à la mutation R77C dans l'a-sarcoglycane et en présence des inhibiteurs 5-azacytidine (ou 5-aza, un inhibiteur de la méthylation de l'ADN) et Saha (pour « SuberoylAnilide Hydroxamic Acid », un inhibiteur des histones désacétylases).
Figure 1 : Marquage immunohistochimique de la protéine α-sarcoglycane (a-SG) dans des cellules humaines HER 911 quadri-transfectées :
   A/ à l'aide de l'α-SG non mutée
   B/ à l'aide de l'α-SG portant la mutation R77C
   C/ à l'aide de l'α-SG portant la mutation R77C et en présence de 5-aza 5 µM.
Figure 2 : Marquage immunohistochimique de la protéine α-sarcoglycane (a-SG) dans des cellules humaines HER 911 quadri-transfectées :
   A/ à l'aide de l'α-SG non mutée
   B/ à l'aide de l'α-SG portant la mutation R77C
   C/ à l'aide de l'α-SG portant la mutation R77C et en présence de SAHA 1 µM.

### 1/MATERIELS ET METHODES

### 1.1 Cultures cellulaires, transfections et traitements

Des cellules HER-911 ("Human Embryonic Retinoblast") ont été ensemencées dans des flacons et cultivées jusqu'à environ 80% de confluence dans du milieu Dulbecco's modified Eagle's + GlutaMAX (Gibco, Invitrogen) contenant 10% de sérum de veau foetal, 0.01 mg/ml de gentamicine et 1% MEM Non Essential Amino Acids (Gibco, Invitrogen). Les cellules ont été maintenues dans une atmosphère humidifiée à 5% en CO₂ et à 37°C.

Les cellules HER-91 ont été transfectées à l'aide de 12 µl de Fugene.6 Transfection Reagent (Roche) pour 2 µg de plasmide (0.5 µg de chacune des 4 constructions sarcoglycane (SG) pour cellules HER-911). Les plasmides exprimant les SG, pcDNA3.V5-his_hα-SG, pcDNA3.V5-his_hβ-SG, pcDNA3.V5-his_hy-SG et pcDNA3.V5-his_hδ1-SG (variant transcriptionnel de type 1), sont des plasmides où l'expression de la séquence codante des sarcoglycanes respectives est sous le contrôle du promoteur CMV.

Après 48h, les cellules ont été traitées pendant 24h avec 5 µM de 5-aza-2'-deoxycytidine ou 1 µM de SAHA (Suberoylanilide hydroxamic acid).

### 1.2 Immunofluorescence

Les cellules HER-911 ont été fixées par incubation avec 3.7% de formaldéhyde pendant 15 min à temperature ambiante. Si nécessaire, une perméabilisation a été réalisée en utilisant du Triton X-100 0.2% pendant 20 min. Après avoir été saturées avec du sérum de veau foetal à 20% pendant 30 min, les cellules ont été incubées avec des anticorps primaires à des dilutions adaptées (dans du PBS) pendant 1h à temperature ambiante. Un anticorps murin monoclonal spécifique pour α-SG (NCL-A-SARC) est disponible auprès de Novocastra. Les cellules ont été lavées 3 fois avec du PBS puis incubées 1h avec des anticorps secondaires dilués au 1/1000 dans du PBS. Enfin, les cellules ont été lavées dans du PBS, montées en utilisant un milieu Vectashield Mounting Medium avec du DAPI (Vector, H-1200), puis examinées avec un microscope confocal à immunofluorescence (Leica).

### 2/ RÉSULTATS

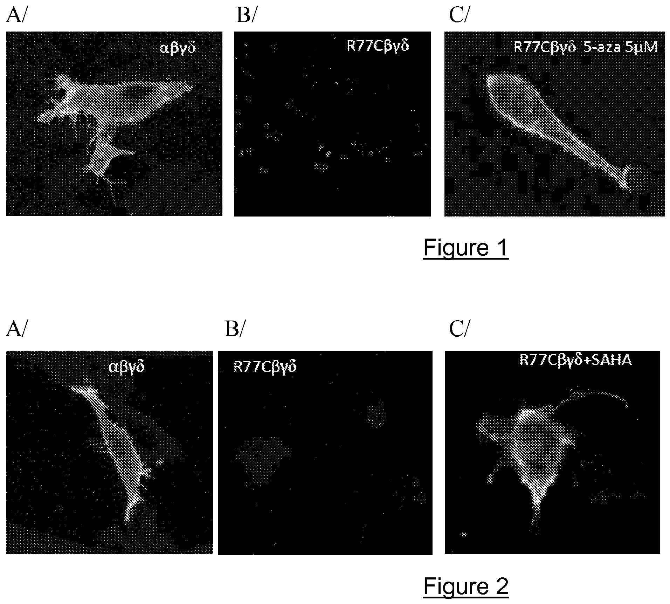

Des cellules humaines HER 911 ont été quadri-transfectées avec les plasmides codant pour les sarcoglycanes β, γ et δ et un plasmide codant soit pour l'a-sarcoglycane non mutée (Fig. 1A et Fig. 2A), soit pour l'a-sarcoglycane mutée R77C (Fig. 1B et 1C ; Fig. 2B et 2C).

Un immuno-marquage a ensuite été réalisé sur les cellules non perméabilisées en utilisant un anticorps dirigé contre la partie extracellulaire de l'a-sarcoglycane, ce qui permet de visualiser la protéine uniquement au niveau de la membrane cellulaire et donc de comparer l'adressage de la protéine mutée avec celui de la protéine normale.

Contrairement à la forme non mutée de la protéine α- sarcoglycane (Fig. 1A et 2A), la forme mutée R77C de cette protéine n'est pas adressée à la membrane des cellules HER 911 (Fig. 1B et 2B). Le traitement des cellules par la 5-azacytidine (Fig. 1C) ou par le SAHA (Fig. 2C), restaure la localisation membranaire de la protéine mutée R77C.

Le principe sous-tendant la présente invention a donc été confirmé *in vitro* sur un modèle de cellules quadritransfectées permettant de reconstituer le complexe des sarcoglycanes, en analysant l'adressage membranaire du complexe. L'utilisation de drogues modifiant l'épigénome, à savoir la 5-azacytidine, un inhibiteur de la méthylation de l'ADN ou Saha, un inhibiteur des histones désacétylases, a permis la reconstitution du complexe à la membrane.

### BIBLIOGRAPHIE

Ben Othmane K, Speer MC, Stauffer J, Blel S, Middleton L, Ben Hamida C, Etribi A, Loeb D, Hentati F, Roses AD, "Evidence for linkage disequilibrium in chromosome 13-linked Duchenne-like muscular dystrophy (LGMD2C)." Am J Hum Genet. 1995 Sep;57(3):732-4.
Bushby, K.M., and J.S. Beckmann, 1995, "The limb-girdle muscular dystrophies-- proposal for a new nomenclature". Neuromuscul Disord. 5:337-43.
Daniele, N., I. Richard, and M. Bartoli, 2007, "Ins and outs of therapy in limb girdle muscular dystrophies." Int JBiochem Cell Biol. 39:1608-24.
Duclos, F., V. Straub, S.A. Moore, D.P. Venzke, R.F. Hrstka, R.H. Crosbie, M. Durbeej, C.S. Lebakken, A.J. Ettinger, J. van der Meulen, K.H. Holt, L.E. Lim, J.R. Sanes, B.L. Davidson, J.A. Faulkner, R. Williamson, and K.P. Campbell, 1998, "Progressive muscular dystrophy in alpha-sarcoglycan-deficient mice." J Cell Biol. 142:1461-71.
Egelhofer et al., Nat. Struct. Mol. Biol. 2011 Jan ; 18(1) : 91-3.
Kefi M, Amouri R, Driss A, Ben Hamida C, Ben Hamida M, Kunkel LM, Hentati F. "Phenotype and sarcoglycan expression in Tunisian LGMD 2C patients sharing the same del521-T mutation Neuromuscul Disord." 2003 Dec;13(10):779-87.
Lim LE, Duclos F, Broux O, Bourg N, Sunada Y, Allamand V, Meyer J, Richard I, Moomaw C, Slaughter C, 1995, "Beta-sarcoglycan: characterization and role in limb-girdle muscular dystrophy linked to 4q12." Nat Genet. Nov;11(3):257-65.
Nigro, V., 2003, "Molecular bases of autosomal recessive limb-girdle muscular dystrophies." Acta Myol. 22:35-42.
Passos-Bueno MR, Richard I, Vainzof M, Fougerousse F, Weissenbach J, Broux O, Cohen D, Akiyama J, Marie SK, Carvalho AA, 1993, "Evidence of genetic heterogeneity in the autosomal recessive adult forms of limb-girdle muscular dystrophy following linkage analysis with 15q probes in Brazilian families. » J Med Genet. May;30(5):385-7.
Passos-Bueno MR, Moreira ES, Vainzof M, Marie SK, Zatz M., 1996, "Linkage analysis in autosomal recessive limb-girdle muscular dystrophy (AR LGMD) maps a sixth form to 5q33-34 (LGMD2F) and indicates that there is at least one more subtype of AR LGMD." Hum Mol Genet. Jun;5(6):815-20.
Piccolo, F., M. Jeanpierre, F. Leturcq, C. Dode, K. Azibi, A. Toutain, L. Merlini, L. Jarre, C. Navarro, R. Krishnamoorthy, F.M. Tome, J.A. Urtizberea, J.S. Beckmann, K.P. Campbell, and J.C. Kaplan, 1996, "A founder mutation in the gamma-sarcoglycan gene of gypsies possibly predating their migration out of India." Hum Mol Genet. 5:2019-22.
Todorova A, Ashikov A, Beltcheva O, Tournev I, Kremensky I. "C283Y mutation and other C-terminal nucleotide changes in the gamma-sarcoglycan gene in the Bulgarian Gypsy population." Hum Mutat. 1999;14(1):40-4. Erratum in: Hum Mutat 2000;15(5):479.

## Revendications

1. Composition pharmaceutique comprenant au moins un composé choisi parmi:
- un inhibiteur de la méthylation de l'ADN, avantageusement un inhibiteur des méthyltransférases ; ou
- un inhibiteur des histones désacétylases,
pour utilisation dans le traitement des maladies génétiques musculaires causées par une anomalie de conformation d'au moins une protéine, ladite anomalie entraînant la dégradation cellulaire de la protéine, la maladie génétique musculaire étant une dystrophie musculaire progressive, la protéine étant choisie dans le groupe constitué des sarcoglycanes, de la dysferline, de l'anoctamine 5 et de la protéine FKRP (« Fukutin-Related Protein »).

2. Composition pour son utilisation selon la revendication 1, ***caractérisée* en ce que** le composé est le phénylbutyrate, le SAHA ou la 5-azacytidine.

3. Composition pour son utilisation selon l'une quelconque des revendications précédentes, ***caractérisée* en ce que** la dystrophie musculaire progressive est choisie dans le groupe suivant : les sarcoglycanopathies, les dysferlinopathies, les anoctaminopathies et les dystrophies associées à une anomalie de FKRP (« Fukutin-Related Protein »).

4. Composition pour son utilisation selon la revendication 3, ***caractérisée* en ce que** la dystrophie musculaire progressive est une sarcoglycanopathie.

5. Composition pour son utilisation selon la revendication 4, ***caractérisée* en ce que** la sarcoglycanopathie est l'a-sarcoglycanopathie (LGMD2D).

6. Composition pharmaceutique pour son utilisation selon l'une quelconque des revendications précédentes, ***caractérisée* en ce que** la composition est administrée par voie intramusculaire, intrapéritonéale, sous-cutanée, orale ou intraveineuse.

## Patentansprüche

1. Pharmazeutische Zusammensetzung, die mindestens eine Verbindung enthält, die ausgewählt wird aus:
- einem DNA- Methylierungshemmer, vorteilhafterweise ein Methyltransferasehemmer; oder
- einem Histon-Deacetylase-Hemmer,
zur Verwendung bei der Behandlung genetischer Muskelerkrankungen, die mit einer Konformationsstörung mindestens eines Proteins verbunden sind, wobei die Störung den zellulären Abbau des Proteins verursacht, bei dieser genetischen Muskelerkrankung handelt es sich um eine progressive Muskeldystrophie, das Protein wird ausgewählt aus der Gruppe bestehend aus den Sarcoglycanen, Dysferlin, Anoctamin 5 und dem Protein FKRP (« Fukutin-Related Protein »).

2. Zusammensetzung zur Verwendung nach Anspruch 1, **dadurch gekennzeichnet, dass** es sich bei der Verbindung um Phenylbutyrat, SAHA oder 5-azacytidin handelt.

3. Zusammensetzung zur Verwendung nach einem der vorangehenden Ansprüche, **dadurch gekennzeichnet, dass** die progressive Muskeldystrophie ausgewählt wird aus der folgenden Gruppe: Sarcoglycanopathien, Dysferlinopathien, Anoctaminopathien und Dystrophien in Verbindung mit einer FKRP (« Fukutin-Related Protein ») - Störung.

4. Zusammensetzung zur Verwendung nach Anspruch 3, **dadurch gekennzeichnet, dass** es sich bei der progressiven Muskeldystrophie um eine Sarcoglycanopathie handelt.

5. Zusammensetzung zur Verwendung nach Anspruch 4, **dadurch gekennzeichnet, dass** es sich bei der Sarcoglycanopathie um eine α- Sarcoglycanopathie (LGMD2D) handelt.

6. Zusammensetzung zur Verwendung nach einem der vorangehenden Ansprüche, **dadurch gekennzeichnet, dass** die Zusammensetzung auf intramuskulärem, intraperitonalem, subkutanem, oralen oder intravenösem Weg verabreicht wird.

## Claims

1. A pharmaceutical composition comprising at least one compound chosen among:
- an inhibitor of DNA methylation, advantageously an inhibitor of methyltransferases; or
- an inhibitor of histone deacetylases,
for use in the treatment of genetic muscular diseases due to a conformational disorder of at least one protein, said disorder causing the cellular degradation of the protein, wherein the genetic muscular disease is a progressive muscular dystrophy and the protein is selected in the group consisting of: sarcoglycans, dysferlin, anoctamin 5, and the FKRP ("Fukutin-Related Protein") protein.

2. A composition for its use according to claim 1, **characterized in that** the compound is phenylbutyrate, SAHA or 5-azacytidine.

3. A composition for its use according to any of the foregoing claims, **characterized in that** the progressive muscular dystrophy is selected in the group consisting of: sarcoglycanopathies, dysferlinopathies, anoctaminopathies, and dystrophies associated with a FKRP ("Fukutin-Related Protein") disorder.

4. A composition for its use according to claim 3, **characterized in that** the progressive muscular dystrophy is a sarcoglycanopathy.

5. A composition for its use according to claim 4, **characterized in that** the sarcoglycanopathy is α-sarcoglycanopathy (LGMD2D).

6. A pharmaceutical composition for its use according to any of the foregoing claims, **characterized in that** the composition is administered by intramuscular, intraperitoneal, subcutaneous, oral, or intravenous route.
